# EUROPEAN PATENT SPECIFICATION

(11) **EP 1 581 226 B1**
(45) Date of publication and mention of the grant of the patent: **20.02.2008**
(21) Application number: 02783368.0
(22) Date of filing: 06.11.2002
(51) Int. Cl.: A61K 31/496, A61K 47/40, A61P 31/06

(54) **INCLUSION COMPLEX OF ANTI-TUBERCULAR RIFAMPICIN WITH BETA-CYCLODEXTRIN OR 2-HYDROXYPROPYL BETA-CYCLODEXTRIN AND A PROCESS FOR PRODUCING THE SAME**
EINSCHLUSSKOMPLEX VON TUBERKULOSTATISCHEM RIFAMPICIN MIT BETA-CYCLODEXTRIN ODER 2-HYDROXYPROPYL BETA-CYCLODEXTRIN UND EIN PROZESS ZUR HERSTELLUNG DESSELBEN
INCLUSION D'UNE RIFAMPICINE ANTI-TUBERCULAIRE AVEC UNE BETA-CYCLODEXTRINE OU UNE 2-HYDROXYPROPYL BETA-CYCLODEXTRINE ET SON PROCEDE D'OBTENTION

(43) Date of publication of application: 05.10.2005
(73) Proprietor: COUNCIL OF SCIENTIFIC AND INDUSTRIAL RESEARCH, New Delhi 110 001 (IN)
(72) Inventor: RAO, Kakulapati, Rama, Indian Inst. of Chem. Techn, Andhra Pradesh 500 007 (IN); BHANUMATHI, Nanduri, Indian Inst of Chemical Techn, Andhra Pradesh 500 007 (IN); YADAV, Jhillu Singh, Indian Inst of Chemical Techn, Andhra Pradesh 500 007 (IN); KRISHNAVENI, Neelam S., Indian Inst of Chem. Techn, Andhra Pradesh 500 007 (IN)
(74) Representative: Ekström, Nils
(86) International application number: PCT/IB2002/004705
(87) International publication number: WO 2004/041284

(56) References cited:
- WO-A-00/54751
- WO-A-02/32459
- KUCHEKAR, B. S. ET AL: "Solid dispersions of rifampicin" EASTERN PHARMACIST ( 1998 ), 41(492), 133-134 , XP001145510
- DATABASE WPI Section Ch, Week 199105 Derwent Publications Ltd., London, GB; Class B04, AN 1991-031971 XP002232706 -& JP 02 300140 A (KYORIN PHARM CO LTD), 12 December 1990 (1990-12-12)
- RAJEWSKI R A ET AL: "PHARMACEUTICAL APPLICATIONS OF CYCLODEXTRINS. 2. IN VIVO DRUG DELIVERY" JOURNAL OF PHARMACEUTICAL SCIENCES, AMERICAN PHARMACEUTICAL ASSOCIATION. WASHINGTON, US, vol. 85, no. 11, 1 November 1996 (1996-11-01), pages 1142-1169, XP000629515 ISSN: 0022-3549

## Description

### Technical Field

The present invention relates to an inclusion complex comprising Rifampicin and cyclodextrin useful as drug in tuberculosis. The present invention also relates to synthesis of Rifampicin- cyclodextrin inclusion complexes, which find use in tuberculosis therapy as drug delivery systems.

### Background Art

Rifampicin is the international nonproprietary name and other names used are Rifamycin AMP, Rifampin and Rifaldazine. Rifampicin is designated by IUPAC rules as 2,7-(epoxypentadeca[1,11,13]trienimino)naphtho[2,1-b]furan 1, 11(2H)-dione 5,6,9,17,19,21-hexa hydroxy-23-methoxy-2,4,12,16,18,20,22-hepta methyl-8-[N-(4-methyl-1-piperazinyl)formimidoyl]-21-acetate.

Cyclodextrins (CDs) are cyclic oligosaccharides possessing hydrophobic cavities. CDs can be used in drugs either for complexation or as auxiliaries such as diluents, solubilisers or tablet ingredients (Comprehensive Supramolecular Chemistry, Vol 3, Szejtli J, Osa T, Pergamon, UK, 1996). The advantage of using CDs mainly comes from their inclusion complex formation. The complexation can protect the molecule and can eventually have considerable pharmaceutical potential. Various advantageous effects of inclusion complex formation are as follows.
i) Incompatible drugs can be mixed when one of them is complexed with CDs.
ii) The release rate of drugs can be controlled.
iii) The solubility of water insoluble drugs can be improved.
iv) The instability of drugs in water / acidic stomach conditions can be improved as the rate of hydrolysis, photo decomposition, auto catalytic reactions etc. are considerably reduced.
iv) Percutaneous or rectal absorption can be improved by the enhanced release of drugs from ointments or suppository bases. Thus, CD- inclusion complexes of drugs have several advantages.

The inclusion complex formation can be identified by powder X-ray diffraction patterns and IR spectroscopy (Comprehensive Supramolecular Chemistry, Vol 3, Szejtli J, Osa T, Pergamon, UK, 1996).

The recent finding (Chronicle Pharmabiz, p.28, Dec.20, 2001) of impaired bioavailability of Rifampicin in the presence of Isoniazid in fixed dose combinations (FDCs) due to decomposition of Rifampicin in the stomach before it is absorbed into the body has prompted us to make inclusion complexes of Rifampicin with β-cyclodextrin and (2-hydroxypropyl)-β-cyclodextrin and characterize them. They have the potential to be used as new drug delivery systems for stability and slow release. However, the combinations reported so far are only dispersions of Rifampicin and cyclodextrin (East. Pharm., p.133, vol. 41(492), 1998), but the inclusion complexes have not yet been isolated and characterized.

Kuchekar et al. ("Solid dispersions of rifampcin", Eastern Pharmacist (1998) 41(492), 133-134) discloses a process of wet mixing rifampicin and cyclodextrin in chloroform followed by evaporation of the solvent.

WO 02/32459 discloses host-guest complexes of rifampicin with cyclodextrin that are produced by physical mixing.

WO 00/54751 discloses complexes of rifampicin with cyclodextrin produced from a plasticized mixture of both substances.

JP 02300140 discloses forming of clathrate complexes between e.g. glyceofulvin and cyclodextrin.

Accordingly, studies were undertaken to make the inclusion complexes of Rifampicin with β-cyclodextrin (β-CD) and 2-hydroxypropyl-β-cyclodextrin (HP-β-CD).

### Objects of the invention

The main object of the invention is to establish a inclusion complex comprising Rifampicin and cyclodextrins for the treatment of tuberculosis.

Another object of the present invention, is to establish a process for the formation of Rifampicin with β-CD or HP-β-CD for possible use as drug delivery system.

Another object of the present invention is to establish a process for the formation of inclusion complexes of cyclodextrins with large size molecules.

### Brief description of the accompanying drawings

In the drawings accompanying this specification,

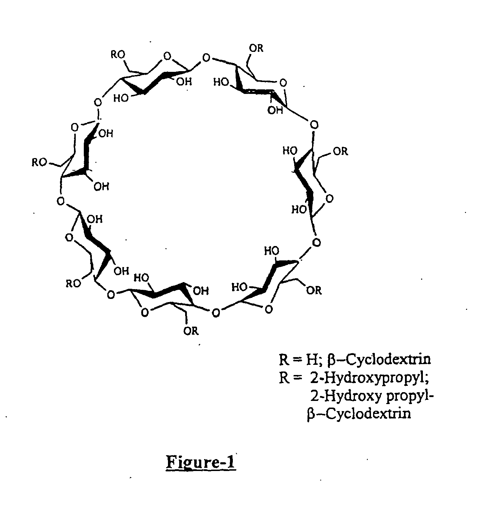

**Fig 1** represents the structures of β-cyclodextrin and (2-Hydroxypropyl) - β-cyclodextrin.

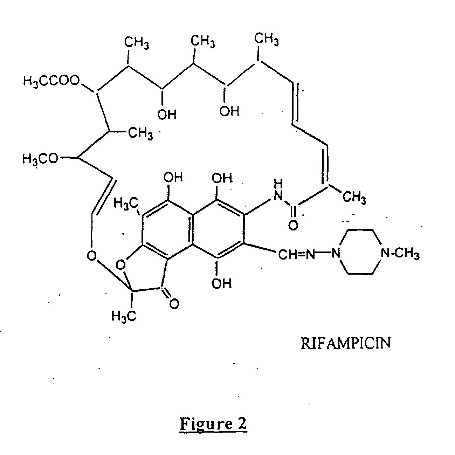

**Fig 2** represents Rifampicin.

### Summary of the invention

The present invention provides a process for preparation of complexes of Rifampicin and cyclodextrin and a product obtainable by such a process. In addition, the present invention provides a anti-tubercular drug comprising such a product; such a product for medical use and use of such a product for preparing a pharmaceutical composition for treating tuberculosis.

### Detailed of the Invention

Accordingly, the present invention provides an inclusion complex of Rifampicin with cyclodextrin as an anti-tubercular drug.

The cyclodextrin used is selected from β-cyclodextrin and 2-hydroxy propyl cyclodextrin. In an embodiment, the inclusion complex is characterized by X-ray diffraction and Infrared studies and the inclusion of complex has following physical and chemical characteristics.

Still another embodiment, the inclusion complex enhances the bioavailability and solubility of the drug Rifampicin.

Still another embodiment, the inclusion complex and the drug exist in an encapsulated form leading to controlled release of the drug.

Yet another embodiment, the inclusion complex has an improved stability of Rifampicin in fixed dose combination.

Yet, another embodiment, the inclusion complex of the present invention, gives a new approach to anti-tuberculosis therapy containing fixed dose combination.

One more embodiment of the invention provides a process for preparation of inclusion complexes of Rifampicin with β-cyclodextrin, the said process comprising adding Rifampicin to cyclodextrin and grinding in an agate mortar to form an uniform powdery material of Rifampicin-dextrin inclusion complex.

Another embodiment, the encapsulation of the drug under solid condition is achieved which enhances bioavailability and solubility.

In another embodiment of the invention, provides a process for the preparation of inclusion complexes of Rifampicin with β-cyclodextrin (β-CD) or 2-Hydroxypropyl β-cyclodextrin (HP-β-CD) which comprises a phenomenon of converting a free drug into an encapsulated form under solid state conditions.

The cyclodextrins forming inclusion complexes with Rifampicin an anti TB drug, are β-CD or HP-β-CD.

Still another embodiment, the formation of cyclodextrin complexes with Rifampicin and these are β-CD or HP-β-CD.

The invention provides an inclusion complex of Rifampicin with cyclodextrin as an anti-tubercular drug.

The substrate forming inclusion complex with cyclodextrins is the anti-tubercular drug " Rifampicin".

The cyclodextrins which form inclusion complexes with "Rifampicin" are β-cyclodextrin which is a cyclic oligosaccharide consisting of seven glucose units and 2-Hydroxypropyl-β-cyclodextrin (HP-β-CD) which is a β-cyclodextrin substituted with hydroxypropyl group at 2-position. HP-β-CD has also been used as a drug carrier due to its low toxicity, high tolerance and excellent solubilizing and stabilizing abilities. HP-β-CD has generally been found to be safe and no adverse effects were observed in human studies. (Comprehensive Supramolecular Chemistry, Vol 3, Szejtli J, Osa T, Pergamon, UK, 1996).

As a result of above, an intensive study conducted by the inventors with the aim of achieving the afore mentioned objectives, a process for the synthesis of inclusion complexes of the anti-tubercular drug, Rifampicin with β-cyclodextrin (β-CD) or 2-hydroxypropyl-β-cyclodextrin (HP-β-CD) has been achieved for the first time.

Accordingly, the present invention deals with the synthesis of inclusion complexes of the anti-tubercular drug , Rifampicin with β-CD or HP-β-CD. The synthesis of each compound has been described in detail.

The process involves the inclusion complex formation of Rifampicin with cyclodextrins. The cyclodextrins (Fig-1) are cyclic oligosaccharides possessing hydrophobic cavities and mimic enzymes in their capability to bind substrates selectively and catalyze chemical reactions. β-Cyclodextrin consists of seven glucose units linked by α-1,4 glycosidic bonds into a macrocycle with a hydrophobic cavity. HP-β-CD is a substituted β-CD at 2-position with a 2-hydroxy propyl group. Each cyclodextrin has its own ability to form inclusion complexes with specific guests into the hydrophobic cyclodextrin cavity. The most important pharmaceutical application of cyclodextrins is to enhance the solubility and bioavailability of drug molecules.

The inclusion complexes of the anti-tubercular drug Rifampicin with cyclodextrins were prepared by adding Rifampicin in equimolar ratio to the respective cyclodextrins and intimately grinding the mixture using mortar and pestle for varying reaction times ranging from five to eight hours. The following examples are given by way of illustration and therefore should not construe the limit of the scope of the present invention.

### EXAMPLE 1

### Rifampicin -β- cyclodextrin inclusion complex:

The cyclodextrin inclusion complex was prepared by the grinding method under solid state conditions. β-Cyclodextrin (13.79 g) was taken in an agate mortar and Rifampicin (10g) was added while mixing intimately. The ingredients were continuously ground ranging from 5-8 hrs to form a uniform powdery material. The inclusion complex of "Rifampicin" with β-cyclodextrins thus formed has been characterized by the powder X-ray diffraction patterns and IR spectral data. The inclusion complex has been identified by comparing its X-ray and IR spectral data with Rifampicin and β-CD.

### Powder X-ray Studies:

**Instrument:** Powder X-ray Diffractometer, Siemens / D-5000
The powder X-ray diffractograms were measured in 2θ angles.
The most significant measurements are as follows:

### β-CD:

4.3, 6.2, 8.9, 10.4, 12.6, 18.6, 22.6, 27.0, 35.2

### Rifampicin:

7.8, 9.5, 10.9, 12.6, 15.8, 16.9, 19.6, 21.3, 26.0

### Rifampicin--β-cyclodextrin complex (Rif- β- CD):

4.3, 8.7, 10.6, 12.6, 15.7, 18.8, 25. 5, 35.2, 46.4

In the inclusion complex, some significant peaks are either shifted, disappeared or some new peaks have appeared. The peaks at **18.8** and **22.6** in β-CD have disappeared in the complex. The peaks at **4.3, 12.6** and **27.0** in β- CD have been reduced in intensity in the complex. The new peaks that appeared in the complex are at **18.6, 25.5** and **46.4** Similar comparison of data with Rifampicin is as follows.

The following peaks of Rifampicin that disappeared in the complex are **9.5, 10.9, 19.6** and **26.0.** The significant peaks of Rifampicin at **12.6, 15.8, 16.9** and **21.3** are reduced in intensity.

### Infrared spectral studies:

**Instrument:** Perkin Elmer Spectrum RX /Ft IR system 500-3500 cm⁻¹

The IR spectra of the drug Rifampicin complex with β-CD and also the individual drug Rifampicin have been recorded as KBr pellets.

The inclusion complex formation has also been proved by IR spectroscopy. Bands due to the included part of the guest molecule have shifted or their intensities altered. The acetoxyl C=O vibration at **1728.2 cm⁻¹** and carbonyl C=O absorption at **1730.4 cm⁻¹** of Rifampicin have been shifted to lower frequency and appear as single peak at**1722.2 cm⁻¹** where as the amide NH-C=O shows only a minor shift from **1651.2 cm⁻¹ to 1647.8cm**⁻¹. However, only a small shift was observed for C=C vibration from **1566.4 cm⁻¹** in the drug to **1565.1cm⁻¹** in the complex .This clearly indicates the formation of inclusion complex of Rifampicin with β-CD.

### EXAMPLE 2

### Rifampicin -2-Hydroxypropyl -β- cyclodextrin inclusion complex:

To Rifampicin (10 g) in an agate mortar, 2- hydroxypropyl -β-cyclodextrin (16.77 g) was added and ground well for periods ranging from 5 to 8 hrs to form an uniform powdery material. The inclusion complex of the drug thus formed was isolated and characterized.

### Powder X-ray studies:

Rifampicin with 2- hydroxypropyl -β-cyclodextrin (HP-β-CD) complex has been confirmed by comparing its data of X-ray diffraction pattern with the parent drug and HP-β-CD.
The important peaks are shown hereunder.

### 2-HP-β-CD:

4.8, 11.6, 17.4, 19.1, 23.1, 29.1, 33.0, 35.0, 39.9
Rifampicin-2-hydroxy propyl-β-cyclodextrin inclusion complex (**Rif- 2HP-** β-**CD):** 1.4, 5.9, 12.8, 14.2, 16.3, 18.2, 21.4, 25.8, 30.6 and 31.8

Comparison of the data of the complex with Rifampicin and HP-β-CD are as follows. The following peaks of Rifampicin at **7.8, 9.5** and **10.9** have disappeared in the complex. The peak at **21.3** was reduced in intensity as compared to Rifampicin. As compared to HP-β-CD, new peaks have appeared at **14.2, 25.8** and disappearance of the peaks at **4.8** and **11.6** was observed. The peak at **23.1** was reduced in intensity.

Thus the difference in the X-ray diffraction patterns of the inclusion complexes of the drug Rifampicin with β-CD and HP-β-CD and that of the individual components by the appearance of new peaks, disappearance of some peaks and also reduction in intensity of some more peaks as described above clearly indicates the formation of inclusion complex of Rifampicin with β-CD and HP-β-CD.

### Infrared spectral studies:

Infrared spectral studies have also been carried out to confirm the formation of inclusion complex.

The IR spectrum of RIF-HP-β-CD complex shows the merging of the acetoxyl C=O at **1728.2 cm⁻¹** and carbonyl C=O absorption at **1730.4 cm⁻¹** of Rifampicin to give a single peak at a lower frequency **1719.8 cm⁻¹,** whereas the amide NH-C=O absorption of Rifampicin at **1651.2 cm⁻¹** shifts to a lower frequency at **1648.4 cm⁻¹.** A significant shift in C=C absorption band from **1566.4 cm⁻¹** to **1562.8 cm⁻¹** has also been observed. This data clearly indicates the formation of inclusion complex of Rifampicin with HP-β-CD.

### The main advantages of the present invention are:

1) The inclusion complex of Rifampicin with β-CD and HP-β-CD protects the drug and this can have considerable pharmaceutical potential.
2) It may be possible to control the release rate of the anti-tubercular drug, Rifampicin.
3) There is also a possibility of improving the stability of Rifampicin in fixed dose combinations (FDCs)
4) This invention may give new approach to anti-tuberculosis therapy containing FDCs.

## Claims

1. Process for preparation of complexes of Rifampicin and cyclodextrin, comprising the step of:
- preparing a mixture of Rifampicin and cyclodextrin,
**characterized in**
**that** the process further comprises the step of:
- grinding, under solid state conditions, the mixture during 5-8 hours as to form a uniform powdery material,
wherein the mixture contains equimolar ratios of Rifampicin and cyclodextrin,
wherein the grinding step includes the use of mortar and pestle, and
wherein the cyclodextrin is β-cyclodextrin.

2. Process according to claim 1, wherein the β-cyclodextrin is 2-hydroxypropyl-β-cyclodextrin.

3. Product comprising complexes of Rifampicin and cyclodextrin,
**characterized in**
**that** it is obtainable by a process according to claims 1-2.

4. Anti-tubercular drug comprising a product according to claim 3.

5. A product according to claim 3, for medical use.

6. Use of a product according to claim 3 for preparing a pharmaceutical composition for treating tuberculosis.

## Patentansprüche

1. Verfahren zur Herstellung von Komplexes von Rifampicin und Cyclodextrin, umfassend den Schritt:
- Herstellen einer Mischung von Rifampicin und Cyclodextrin,
**dadurch gekennzeichnet, dass**
das Verfahren außerdem den Schritt umfaßt:
- Mahlen der Mischung unter den Bedingungen des Festkörperzustands während 5-8 Stunden, um ein einheitliches pulverförmiges Material zu bilden,
wobei die Mischung äquimolare Anteile von Rifampicin and Cyclodextrin enthält,
wobei der Mahlschritt die Verwendung von Mörser and Pistill einschließt, und
wobei das Cyclodextrin β-Cyclodextrin ist.

2. Verfahren nach Anspruch 1, wobei das β-Cyclodextrin 2-Hydroxypropyl-βcyclodextrin ist.

3. Produkt, umfassen Komplex von Rifampicin und Cyclodextrin,
**dadurch gekennzeichnet, dass**
es durch ein Verfahren nach den Ansprüchen 1-2 erhältlich ist.

4. Antituberkulöses Arzneimittel, umfassend ein Produkt nach Anspruch 3.

5. Produkt nach Anspruch 3 zur medizinischen Verwendung.

6. Verwendung eines Produkts nach Anspruch 3 zum Herstellen einer pharmazeutischen Zusammensetzung zum Behandeln von Tuberkulose.

## Revendications

1. Procédé de préparation de complexes de Rifampicine et de cyclodextrine, comprenant l'étape consistant à :
- préparer un mélange de Rifampicine et de cyclodextrine,
**caractérisé en ce que**
le procédé comprend en outre l'étape consistant à :
- broyer, dans des conditions d'état solide, le mélange pendant 5-8 heures de manière à former un matériau pulvérulent uniforme,
dans lequel le mélange contient des proportions équimolaires de Rifampicine et de cyclodextrin,
dans lequel l'étape de broyage fait appel à l'emploi d'un mortier et d'un pilon, et
dans lequel la cyclodextrine est la β-cyclodextrine.

2. Procédé selon la revendication 1, dans lequel la β-cyclodextrine est la 2-hydroxypropyly-β-cyclodextrine.

3. Produit comprenant des complexes de Rifampicine et de cyclodextrine,
**caractérisé en ce que**
l'on peut l'obtenir par un procédé selon les revendications 1-2.

4. Médicament antituberculeux comprenant un produit selon la revendication 3.

5. Produit selon la revendication 3, à usage médical.

6. Utilisation d'un produit selon la revendication 3 pour préparer une composition pharmaceutique destinée à traiter la tuberculose.
